Europäisches Patentamt

European Patent Office

Office européen des brevets

(11) Publication number: **0 326 989 B1**

# EUROPEAN PATENT SPECIFICATION

(45) Date of publication of patent specification: **30.08.95**   (51) Int. Cl.⁶: **C12Q  1/04**, C12Q 1/68

(21) Application number: **89101539.8**

(22) Date of filing: **30.01.89**

(54) **Method and kit for bacterial identification using chromosomal DNA.**

(30) Priority: **30.01.88 JP 21098/88**

(43) Date of publication of application:
**09.08.89 Bulletin  89/32**

(45) Publication of the grant of the patent:
**30.08.95 Bulletin  95/35**

(84) Designated Contracting States:
**CH DE FR GB IT LI NL SE**

(56) References cited:
**EP-A- 0 235 726
EP-A- 0 235 727
EP-A- 0 281 927
WO-A-84/01174**

**JOURNAL OF CLINICAL MICROBIOLOGY, vol.
26, 1988; pp. 1708-1713/**

**INTERNATL. JOURNAL OF SYSTEMATIC BAC-
TERIOLOGY, vol. 39, 1989; pp. 224-229/**

(73) Proprietor: **KOBAYASHI PHARMACEUTICAL
CO. LTD.
25, Doshomachi 5-chome
Higashi-ku
Osaka (JP)**

(72) Inventor: **Hashimoto, Yasuhiro
6, Muromachi
Gifu-shi
Gifu-ken (JP)**
Inventor: **Fujimura, Katsuyuki
9-68-301, Mukoyutakamachi 2-chome
Amagasaki-shi
Hyogo (JP)**
Inventor: **Ezaki, Takayuki
1175-119, Nishinosho
Gifu-shi
Gifu-ken (JP)**
Inventor: **Yabuuchi, Eiko
19-18, Omiya 4-chome
Asahi-ku
Osaka-shi
Osaka (JP)**

(74) Representative: **Hansen, Bernd, Dr.rer.nat. et
al
Hoffmann, Eitle & Partner
Patentanwälte
Postfach 81 04 20
D-81904 München (DE)**

EP 0 326 989 B1

**Description**

The present invention relates to a method of bacterial identification using chromosomal DNA and to a kit to be employed in the practice of this method.

Bacteria are generally identified by examining some of their phenotipic properties or by detecting antigens specific to the bacteria of interest. With the recent increase in the scope and complexity of the fields in which bacterial identification is required, it has often become difficult to accomplish rapid and precise identification by conventional techniques. Hybridization with a specific probe is known as a technique that can be employed in bacterial identification and detection and the recent improvements that have taken place in recombinant DNA technology have made it possible to produce DNA probe having specificity for certain bacteria (see Japanese Patent Public Disclosure Nos. 61-44000 and 60-110299). These probes are useful for the purpose of detecting bacteria of interest directly from a specimen but are not suitable for application to the routine work of identifying unknown bacterial strains because the need to provide many specific probes and to perform hybridization with each probe adds greatly to the time and cost involved. A method have been reported that performs bacterial identification and detection using a chromosomal DNA probe rather than a specific probe (J. Clin. Microbiol., July 1987, pp. 1239 - 1243). However, this method involves difficulty in differentiating between highly homologous bacterial species and finds only limited use in medical and other fields where differentiation between species of the same bacterial genus is required.

Labelling of DNA in the specimen also involves problems. Since the specimen itself is labelled in the prior art, an extraordinarily high degree of skill is required for clinical technicians to label a sample of nucleic acid at the laboratory. In addition, no convenient and reliable method is available for monitoring the yield of labelling (see Japanese Patent Public Disclosure Nos. 60-201256 and 60-201257).

EP-A-0 235 726 is directed to a method for detecting a microorganism in a test sample by firstly preparing labelled DNA extracted from it. Probes are prepared by immobilizing Single-Stranded DNA derived from known microorganisms, and these probes are contacted under hybridizing conditions with the labelled DNA extracted from the sample to form hybridized labelled DNA. This hybridized DNA is then assayed to detect the label to identify the microorganism.

An object, therefore, of the present invention is to provide a method of bacterial identification that is less time-consuming and costly and which is capable of differentiating between highly homologous bacterial species.

Another object of the present invention is to provide a kit that is useful in the practice of this method and which allows bacterial identification to be performed more easily.

In accordance with the present invention, the chromosomal DNA in a bacterium to be assayed is used as a probe to hybridise with the chromosomal DNA in more than one bacterial species that are suspected to be identical to the bacterium in question and the bacterium of interest is identified by checking its homology (the degree of cross hybridization) with individual suspected species. More specifically, the method of the present invention for identifying the species of a bacterial group comprises the following steps:

identifying a bacterial group to which the species of bacterium to be identified belongs by conventional techniques such as its physiological properties, biochemical properties, gram staining or morphological characteristics;

providing a plurality of containers each of which contains single-stranded chromosomal DNA extracted from a bacterial species immobilized in a solid phase, each container containing single-stranded chromosomal DNA extracted from a different bacterial species belonging to the bacterial group species

extracting chromosomal DNA from the bacterial to be identified, and either denaturing the extracted DNA to form single-stranded DNA which is then labelled with a non-radioactive marker or labelling the extracted DNA with a non-radioactive marker and then denaturing it to form single-stranded DNA;

adding the non-radiolabelled single-stranded DNA to the single-stranded DNA in said containers, performing hybridization and washing the containers to remove the excess labelled DNA; and

assaying the non-radioactive marker to determine the degree of hybridization and identifying the species of bacterium which has the highest degree of hybridization;

characterized in that the containers are individual wells of a microtiter plate on whose inner surfaces the single-stranded chromosomal DNAs are immobilized.

Fig. 1 is a flowsheet describing the process of determining the group to which a bacterium to be assayed belongs and which also shows the kits to be used in identifying individual bacterial groups; and

Fig. 2 is a flowsheet describing the procedures for handling the individual components of the kit of the present invention for identifying a gram-negative bacillus or gram-positive coccus in a sample to be assayed.

In order to perform the method of bacterial identification of the present invention in a clinical field, one must first collect a sample from a patient or some other suitable source. The sequence of steps of this method which are to be performed in practice is described hereinafter, with urine being taken as the sample to be collected.

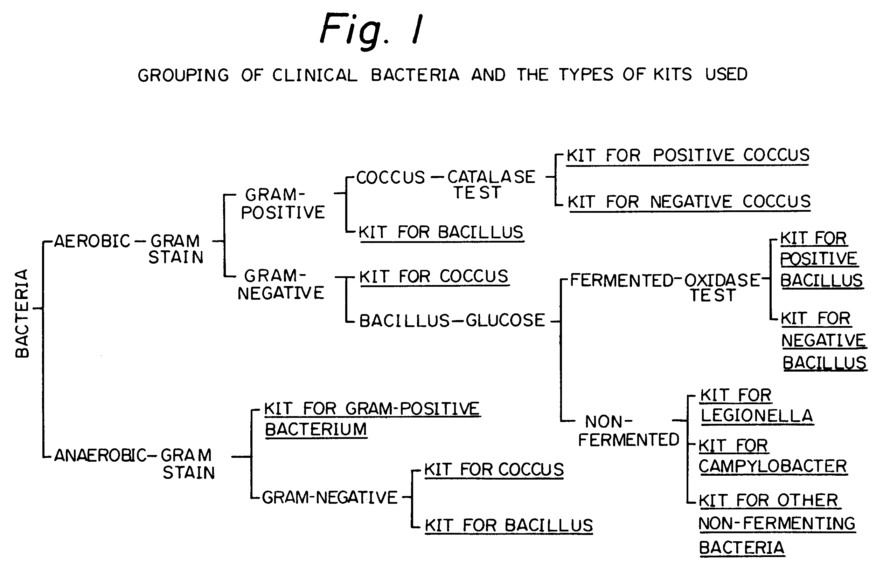

The collected urine is inoculated on a culture medium such as blood agar, BTB medium or phenylethyl alcohol blood agar and subjected to isolation cultivation. The isolated colonies are examined to determine which colony is formed of causative bacterial cells. If the suspected bacteria are aerobic gram-positive cocci, they are subjected to a catalase test. If they are found to be positive to the test, they may be classified in a group generally consisting of the genus Staphylococcus. If the test result is negative, they may be classified in a group generally consisting of the genus Streptococcus. An outline of this grouping of bacteria is shown in Fig. 1. Criteria for grouping are those which are conveniently relied upon at every laboratory, such as physiological properties, biochemical properties, gram staining and morphological characteristics. Bacteria devided into groups may be assayed with appropriate identifying kits.

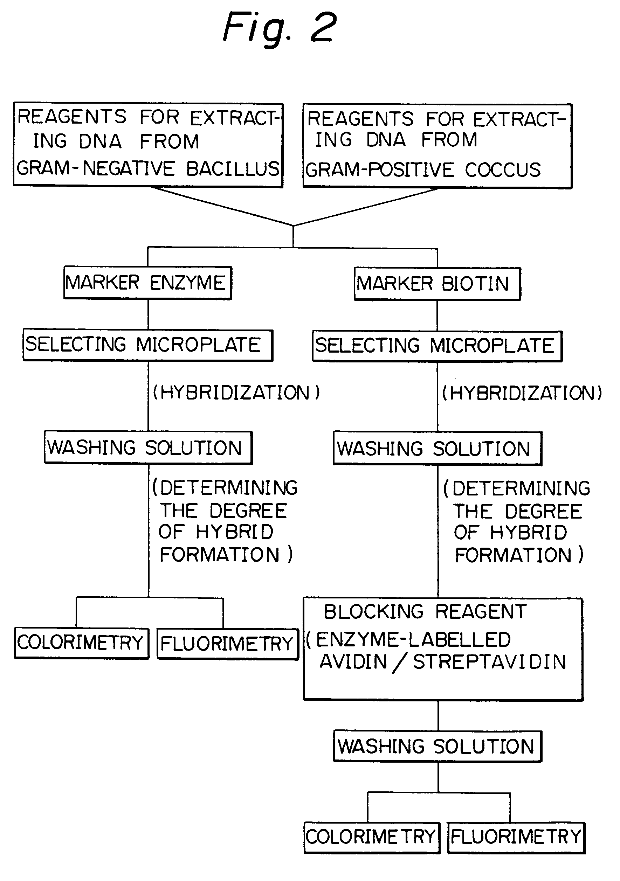

Therefore, the present invention also provides a kit for bacterial identification that contains reagents for extracting DNA from bacterial cells to be assayed and containers in which single-stranded chromosomal DNAs are immobilized in a solid phase for each bacterial group. The amounts of the DNA extracting reagents in the kit of the present invention are several hundredths of those of conventional reagents, so the bacterium to be assayed may be used in a very small amount (or cell count). Conventionally, wet cell weights of 1 - 2 g have been necessary to obtain the DNA to be labelled but the kit of the present invention enables satisfactory assay even if the wet cell weight is only about 5 mg (e.g., 1.5 ml of a cell solution with a turbidity of McFarland No. 3). Even smaller amounts of bacteria will suffice, depending on the group in which they belong. The other constituent element of the kit of the present invention is the containers in which single-stranded chromosomal DNAs are to be immobilized in a solid phase. Microplate wells are appropriate as such containers. If, as a result of preliminary testing, the number of suspected bacteria is reduced to a number below the number of wells, the wells that are not necessary for hybridization may be omitted. Therefore, use of a microplate with detachable wells is convenient and should be included within the scope of the kit of the present invention. The procedures for using the kit of the present invention are illustrated in Fig. 2 with reference to the case where DNAs extracted with one of two sets of DNA extracting reagent, one for extracting DNA from gram-negative bacilli and the other for extracting DNA from gram-positive cocci, are labelled with POD or biotin to prepare labelled DNAs for use in assay.

More specific information on the kit including details of the respective reagents and non-radioactive markers is found in Examples 4 - 7 given hereinafter.

The method of the present invention has the advantage that only one substance need be labelled, namely, the chromosomal DNA from the bacterium to be assayed. Consequently, the method can be completed by a single step of cross-hybridization on a multi-well container such as a microplate.

Bacterial identification by the method of the present invention is based on a comparison of the degree of cross-hybridization, so a single-stranded chromosomal DNA suffices as the DNA to be immobilized in a solid phase and there is no particular need to prepare from the chromosomal DNA fragments of the DNA which is specific to the bacteria of interest. As a consequence, the method of the present invention can be practiced in a very efficient and inexpensive way.

The marker for labelling the DNA to be assayed by the method of the present invention is selected from among non-radioactive markers and can be handled without requiring any sophisticated skill, training or facilities, and this lessens the burden on operators in terms of both time and cost.

Because of the three features described above, it is believed that the method of the present invention enables bacterial identification to be accomplished in a precise and rapid manner.

The kit of the present invention does not employ membrane filters of the kind which have conventionally been used in bacterial taxonomy for determining the homology of two bacteria such as nitrocellulose filters. Instead, the kit uses a solid-phase container and enables determination of bacterial homology by a non-isotopic technique. The solid-phase container may be made of any suitable material such as polystyrene or polyvinyl chloride but in order to accomplish the intended determination of bacterial homology by a non-isotopic technique, it is advisable to use a material that is free from the problems encountered with the use of nitrocellulose filters, such as dissolution of pigments or dyes into spots during determination of enzymatic activity, increased background due to the non-specific adsorption of dyes or pigments on the filter, and difficulties in cleaning.

Since the kit of the present invention is made up of very small amounts of components including the reagents for extracting DNA from the bacterium of interest, the present invention offers the added advantage that the intended assay can be accomplished satisfactorily even if the amount of bacterial cells to be assayed is only several hundredths the magnitude of the levels previously required.

Bacterial assay can be performed in a more convenient and reliable way by employing this kit in the already described method of bacterial identification of the present invention.

The following examples are provided for the purpose of further illustrating the present invention but are in no way to be taken as limiting.

Example 1

(1) Preparing containers in which chromosomal DNAs are immobilized in solid phase

DNA was extracted from 13 species of the genus Legionella by the Marmur method described in "Chemistry of Nucleic Acids: Part I" in A Course in Experimental Biochemistry, Vol. 2, compiled by The Japanese Biochemical Society and published by Tokyo Kogaku Dojin. The extracted DNAs were heated at 100°C for 5 minutes and quenched in ice water to prepare single-stranded DNAs. Each of the single-stranded DNAs was adjusted to a concentration of 10 $\mu$g/m$\ell$ with PBS (pH 7.0) containing 0.1 M MgCl$_2$. The so prepared solutions of DNA from the 13 bacterial species were added to a microplate (Immunoplate II of Nunc Inc. or MicroFluor Black Plate of Dynatech Laboratories, Inc.) in amounts of 100$\mu\ell$/well.

After standing at 35°C for 1 hour, the supernatant was removed and a prehybridization solution [for its recipe, see below under (2)] was added in an amount of 300 $\mu\ell$/well. After standing at 35°C for 1 hour, the supernatant was removed and the residue was stored at 5°C under drying conditions.

(2) Preparing non-radiolabelled single-stranded DNA from specimen

Method 1

DNA was extracted by the Marmur method from a bacterium of the genus Legionella isolated from the human pleural effusion. The extracted DNA was heated at 100°C for 5 minutes and quenched in ice water to prepare a single-stranded DNA. This single-stranded DNA was dissolved at a concentration of 500 $\mu$g/m$\ell$ with 0.1 x SSC, or a 10-fold dilution of a 0.15 M NaCl-0.015 M trisodium citrate solution. A portion (20 $\mu\ell$) of this solution was mixed with 20 $\mu\ell$ of Labezyme-POD (a marker peroxidase produced and sold by Wako Pure Chemicals Industries, Ltd.). After addition of 5% glutaraldehyde (6 $\mu\ell$), the mixture was incubated at 37°C for 10 minutes to prepare a POD labelled DNA. This was mixed with 10 m$\ell$ of a prehybridization solution [0 - 50% formamide; 1 x Denhardt's solution {0.02% BSA, 0.02% polyvinylpyrrolidone (mol. wt. 3.6 x $10^3$ - 4 x $10^4$) and 0.02% Ficoll (mol. wt. 4 x $10^5$)}; 2 x SSC or 0.15 M NaCl-0.03 M Tris-HCl buffer solution; 0 - 6% polyethylene glycol 6000; and 50 - 200 $\mu$g/m$\ell$ of denatured salmon sperm DNA to prepare a hybridization solution.

Method 2

Ten microliters of the single-stranded DNA solution prepared as in Method 1 was mixed with 5 $\mu\ell$ of PHOTOPROBE TM Biotin (Vector Laboratories, Inc.) and the mixture was illuminated for 15 minutes in ice water under a sunlamp placed, 10 cm above. After illumination, the volume of the mixture was adjusted to 100 $\mu\ell$ with a 0.1 M Tris-HCl (pH 9.0) buffer solution. After extraction with 100 $\mu\ell$ of 2-butanol, the 2-butanol layer in the supernatant was removed, followed by another cycle of extraction with 100 $\mu\ell$ of 2-butanol and removal of the supernatant. The aqueous layer was mixed with 10 m$\ell$ of a prehybridization solution to prepare a hybridization solution.

(3) Hybridization and determination of the degree of hybrid formation

In Method 1

The hybridization solution prepared in (2) was added in 100 $\mu\ell$/well to the microplate (Immunoplate II) preconditioned in (1), and reaction was allowed to proceed at 35°C for 2 hours. The supernatant was removed and the wells were washed three times with 2 x SSC solution. After adding 100 $\mu\ell$ of a 3,3',5,5'-tetramethylbenzidine-H$_2$O$_2$ solution, the mixture was incubated for 5 minutes for color development. The reaction was quenched by adding 100 $\mu\ell$ of 2 N HCl and absorbance measurement was measured at $\lambda$ = 450 nm with a microplate reader.

4

In Method 2

The hybridization solution prepared in (2) was added in 100 $\mu\ell$/well to the microplate (MicroFluor Black Plate) preconditioned in (1), and reaction was allowed to proceed at 42°C for 12 hours. The supernatant was removed and the wells were washed three times with 2 x SSC. Thereafter, 300 $\mu\ell$ of PBS containing 2% BSA and 0.1% Triton X-100 was added and the mixture was left to stand at 35°C for 10 minutes. The supernatant was removed and $\beta$-galactosidase-streptavidin(Zymed Laboratories) diluted to 1:1000 with the PBS described above was added in 100-$\mu\ell$ portions. After reaction for 30 minutes at 35°C, the wells were washed three times with PBS containing 0.1% Triton X-100, followed by addition of 4-methylumbelliferyl-$\beta$-D-galactoside solution in 100-$\mu\ell$ portions. Thirty minutes later, the intensity of fluorescence was measured at 450 nm with excitation at 360 nm.

Experimental Results

The results of the experiment conducted by procedures (1) to (3) are described in Table 2.

Table 2

| Bacterial source of DNA fixed in solid phase in microwells | Method 1 (Abs. λ = 450 nm) | Method 2 Intensity of fluorescence |
|---|---|---|
| Legionella pneumophila | 1.21 | 100 |
| Legionella feeleii | 0.33 | <10 |
| Legionella jordanis | 0.20 | <10 |
| Legionella micdadei | 0.25 | <10 |
| Legionella longbeachae | 0.49 | <10 |
| Legionella wadsworthii | 0.46 | <10 |
| Legionella sainthelensi | 0.33 | <10 |
| Legionella hackeliae | 0.40 | 14 |
| Legionella rubrilucens | 0.32 | 10 |
| Legionella cherrii | 0.37 | 10 |
| Legionella dumoffii | 0.44 | <10 |
| Legionella gormanii | 0.45 | <10 |
| Legionella bozemanii | 0.40 | 10 |

The intensity of fluorescence is shown in Table 2 in terms of relative values, with a maximum of 100 being taken as the reference value.

On the basis of the data in Table 2, the bacterium of the genus Legionella isolated from the human pleural effusion was identified as L. pneumophila.

Example 2

(1) When diarrhetic feces were cultivated in Skirrow medium at 42°C for 48 hours under microaerophilic conditions, S type colonies tinged with scarlet grew. These colonies were oxidase positive and did not ferment sugars. These characteristics, combined with the results of gram staining, showed that these colonies were formed of gram-negative bacteria that were in a spirally curved rods, which is characteristic of bacteria of the genus Camplylobacter.

Using a physiological saline-EDTA solution, 1.5 m$\ell$ of a cell suspension having a turbidity of McFarland No. 2 was prepared from the colonies and used as a solution of the bacterium to be tested. This test solution was transferred into an Eppendorf sampling tube and centrifuged at 15,000 rpm for 30 seconds to remove the supernatant. The cells were suspended in 200 $\mu\ell$ of a physiological saline-EDTA solution. A lysozyme powder was dissolved in a dissolving solution and adjusted to a concentration of 5 mg/m$\ell$. A portion (50 $\mu\ell$) of the solution was left to stand at room temperature for 5 minutes. After adding 30 $\mu\ell$ of a 20% SDS (sodium laurylsulfate) solution, the mixture was heated at 60°C for 5 minutes, whereupon it became viscous and translucent.

To this mixture, 200 $\mu\ell$ of phenol-chloroform-isoamyl alcohol (25:24:1) was added, followed by vigorous shaking and centrifugation at 15,000 rpm for 5 minutes. The supernatant was transferred into a new sampling tube and mixed well with 400 $\mu\ell$ of cold ethanol. Following centrifugation at 15,000 rpm for 3 minutes, the supernatant was decanted. The DNA in the precipitate was dissolved in 20 $\mu\ell$ of 0.1 x SSC and 10 $\mu\ell$ of the stock solution was increased to 200 $\mu\ell$ by addition of 0.1 x SSC. The concentration of DNA was determined by absorbance measurement at $\lambda$ = 260 nm with a spectrophotometer. The concentration of DNA in the stock solution was found to be 15 $\mu$g/10 $\mu\ell$. The remaining stock solution was heated at 100°C for 5 minutes and quenched in ice water to prepare single-stranded DNAs. A portion (10 $\mu\ell$) of the solution was mixed with 5 $\mu\ell$ of PHOTOPROBE TM Biotin (Laboratories, Inc.) and the mixture was illuminated for 15 minutes in ice water under a sunlamp placed 10 cm above. After the illumination, the volume of the solution was increased to 100 $\mu\ell$ of 2-butanol and removal of the supernatant. The aqueous layer was mixed with 10 m$\ell$ of a prehybridization solution to prepare a hybridization solution.

(2) The hybridization solution prepared in (1) was added in amounts of 100 $\mu\ell$/well to the Campylobacter microplate preconditioned by the same method as described in Example 1, and reaction was allowed to proceed at 42°C for 12 hours. The supernatant was removed and the wells were washed three times with 2 x SSC. After adding 300 $\mu\ell$ of a blocking reagent, the mixture was left to stand at 35°C for 10 minutes. After removing the supernatant, $\beta$-galactosidase-streptavidindiluted to 1:1000 with a blocking reagent was added in 100 $\mu\ell$ portions, and reaction was allowed to proceed at 35°C for 30 minutes. After washing with a phosphate buffered solution three times, 4-methylumbelliferyl-$\beta$-D-galactoside solution was added in 100-$\mu\ell$ portions. Thirty minutes later, the intensity of fluorescence was measured at 450 nm with excitation at 360 nm.

The result of the experiment described above are summarized in Table 3 in which the intensity of fluorescence is shown in terms of relative values, with a maximum of 100 being taken as the reference value.

Table 3

| Bacterial source of DNA fixed in solid phase in microwells | Intensity of flouorescence |
|---|---|
| Campylobacter fetus | <10 |
| Campylobacter jejuni | 100 |
| Campylobacter coli | 26 |
| Campylobacter pyloridis | <10 |
| Campylobacter sputorum | 12 |

On the basis of the data in Table 3, the bacterium of interest was identified as C. jejuni.

Example 3

(1) A specimen from the human pharyngeal swab was cultivated aerobicallaly on a blood agar plate medium and subjected to both gram staining and a catalase test. Since the colonies were gram-positive, catalase negative, cocoidal bacteria that grew in chains, they were classified in the streptococcus group. Using an EDTA solution, 1.5 m$\ell$ of a cell solution having a turbidity of McFarland No. 3 was prepared from these colonies and used as a solution of the bacterium to be tested. This test solution was transferred into a sampling tube and centrifuged at 15,000 rpm for 30 seconds to remove the supernatant. The cells were suspended in 200 $\mu\ell$ of an EDTA solution. An achromopeptidase powder was dissolved in a dissolving solution, adjusted to a concentration of 5 mg/m$\ell$ and left to stand at room temperature for 5 minutes. After adding 30 $\mu\ell$ of a 20% SDS solution, the mixture was heated at 60°C for 5 minutes, whereupon it became translucent.

To this mixture, 200 $\mu\ell$ of phenol-chloroform-isoamyl alcohol was added, followed by vigorous shaking and centrifugation at 15,000 rpm for 5 minutes. The supernatant was transferred into a new sampling tube and mixed well with 400 $\mu\ell$ of cold ethanol. Following centrifugation at 15,000 rpm for 3 minutes, the supernatant was decanted. The DNA in the precipitate was dissolved in 20 $\mu\ell$ of 0.1 x SSC and 10 $\mu\ell$ of the stock solution was increased to 200 $\mu\ell$ by addition of 0.1 x SSC. The concentration of DNA was determined by absorbance measurement at $\lambda$ = 260 nm with a spectrophotometer. The concentration of DNA in the stock solution was found to be 10 $\mu$g/$\mu\ell$. The remaining stock solution was heated at 100°C for 5 minutes and quenched in ice water to prepare single-stranded DNAs. A portion (10 $\mu\ell$) of the solution was mixed with 5 $\mu\ell$ of PHOTOPROBE TM Biotin (Laboratories, Inc.) and the mixture was illuminated for 15 minutes in ice water under a sunlamp placed 10 cm above. After the illumination, the volume of the solution was increased to 100 $\mu\ell$ with a 0.1 M Tris-HCl (pH 9.0) buffer solution. After extraction with 100 $\mu\ell$ of 2-butanol, 2-butanol in the supernatant was removed, followed by another cycle of extraction with 100 $\mu\ell$ of 2-butanol and removal of the supernatant. The aqueous layer was mixed with 10 m$\ell$ of a prehybridization solution to prepare a hybridization solution.

(2) The hybridization solution prepared in (1) was added in amounts of 100 $\mu\ell$/well to the Campylobacter microplate preconditioned by the same method as described in Example 1, and reaction was allowed to proceed at 42°C for 12 hours. The supernatant was removed and the wells were washed three times with 2 x SSC. After adding 300 $\mu\ell$ of a blocking reagent, the mixture was left to stand at 35°C for 10 minutes. After removing the supernatant, $\beta$-galactosidase-streptavidindiluted to 1:1000 with a blocking reagent was added in 100 $\mu\ell$ portions, and reaction was allowed to proceed at 35°C for 30 minutes. After washing with a phosphate buffer solution three times, 4-methylumbelliferyl-$\beta$-D-galactosidesolution was added in 100-$\mu\ell$ portions. Thirty minutes later, the intensity of fluorescence was measured at 450 nm with excitation at 360 nm.

The result of the experiment described above are summarized in Table 4 in which the intensity of fluorescence is shown in terms of relative values, with a maximum of 100 being taken as the reference value.

Table 4

| Bacterial source of DNA fixed in solid phase in microwells | Intensity of flouorescence |
|---|---|
| Streptococcus mitis | <10 |
| Streptococcus sanguis | 14 |
| Streptococcus oralis | <10 |
| Streptococcus pneumoniae | <10 |
| Spreptococcus mutans | 15 |
| Spreptococcus anginosus | 100 |
| Spreptococcus intermedius | 55 |
| Spreptococcus consetellatus | 52 |
| Spreptococcus salivarius | 19 |
| Spreptococcus morbillorum | <10 |
| Spreptococcus bovis | 17 |
| Spreptococcus acidominimus | <10 |
| Spreptococcus uberis | <10 |
| Spreptococcus parvulus | <10 |
| Spreptococcus pleomorphus | <10 |
| Spreptococcus hansenii | <10 |
| Spreptococcus pyogenes | <10 |
| Spreptococcus agalactiae | <10 |
| Spreptococcus equisimilis | <10 |
| Spreptococcus canis | <10 |
| Spreptococcus equi | <10 |
| Spreptococcus porcinus | <10 |
| Spreptococcus iniae | <10 |
| Spreptococcus suis | <10 |

Example 4

A gram-negative bacillus identifying kit was composed of the following components (1) to (5) in amounts that sufficed for assaying 10 specimens (in Examples 5 - 7, all kit components including reagents and solutions are described on the assumption that they are to be used for assaying 10 specimens).

**(1)  Reagents for extracting DNA from cells**

|   |   |
|---|---|
| (For assaying gram-negative bacilus) | Quantity |
| Physiological saline-EDTA solution | 20 m$\ell$ x 1 |

| | |
|---|---|
| 20% SDS solution | 0.3 mℓ x 1 |
| Lysozyme powder and dissolving solution | 2.5 mg x 1 + 0.5 mℓ x 1 |
| Phenol-chloroform-isoamyl alcohol | 2 mℓ x 1 |
| Ethanol | 4 mℓ x 1 |
| 0.1 x SSC | 5 mℓ x 1 |

(2) <u>Non-radioactive marker</u>

(Marker Biotin)

Manufactured by Biotechnology Research Enterprises S.A. Pty. Ltd. and Vector Laboratories, Inc. and sold under trademarks PHOTO BIOTIN and PHOTOPROBE Biotin, respectively.

(Reagents of marker biotin)

| | |
|---|---|
| PHOTO BIOTIN or PHOTOPROBE Biotin | 0.05 mℓ x 1 |
| 0.1 M Tris-HCl (pH 9.0) | I mℓ x 1 |
| 2-Butanol | 2 mℓ x 1 |
| Prehybridization solution | 100 mℓ x 1 |

(3) <u>Microplate having DNA immobilized in solid phase made of plastic materials such as polystyrene</u>

(4) <u>Washing solution</u>

| | |
|---|---|
| 20 x SSC | 150 mℓ x 1 |

(used as a 10-fold dilution in distilled water)

(5) <u>Reagents for detecting non-radioactive marker</u>

| | |
|---|---|
| Blocking reagent: Phosphate buffer solution containing 2% BSA | 400 mℓ x 1 |
| $\beta$-Galactosidase-streptavidin | 0.1 mℓ x 1 |
| Washing solution: Phophate buffer solution | 150 mℓ x 1 |

(used as a 10-fold dilution in distilled water)

| | |
|---|---|
| Substrate solution: 4-methylumbelliferyl-$\beta$-D-galactoside | 10 mℓ x 1 |
| Diluting solution: Buffer solution containing 1 mM $MgCl_2$ | 100 mℓ x 1 |

(Substrate solution was used as 10-fold dilution in the dilution solution)

The kit composed of the above components (1) - (5) was used in Example 2 and the bacterial species of the genus Campylobacter in the specimen were identified with high efficiency.

Besides the $\beta$-galactosidase-streoptavidin in the kit, streptavidin or avidin labelled with peroxidase or alkaline phosphatase may also be used.

Example 5

A gram-positive coccus identifying kit was composed of the following components (1) - (5).

(1)  Reagents for extracting DNA from cells

(For assaying gram-positive coccus)          Quantity

EDTA solution                                20 mℓ x 1

20% SDS solution                             0.3 mℓ x 1

Achromopeptidase* powder and dissolving

solution                                     2.5 mg x 1 +

                                             0.5 mℓ x 1

Phenol-chloroform-isoamyl alcohol            2 mℓ x 1

Ethanol                                      4 mℓ x 1

0.1 x SSC                                    5 mℓ x 1

* Prepared from Achromobacter lyticus; manufactured and sold by Wako Pure Chemical Industries, Ltd.

(2)  Non-radioactive marker

(Marker biotin)

Manufactured by Biotechnology Research Enterprises S.A. Pty. Ltd. and sold under trademarks PHOTO BIOTIN and PHOTOPROBE Biotin, respectively.

(Reagents of marker biotin)

PHOTO BIOTIN or PHOTOPROBE                    0.05 mℓ x 1

0.1 M Tris-HCl (pH 9.0)                       1 mℓ x 1

2-Butanol                                     2 mℓ x 1

Hybridization solution                       100 mℓ x 1

(3)  Microplate having DNA immobilized in solid phase made of plastic materials such as polystyrene

(4)  Washing solution

20 x SSC                                     150 mℓ x 1

(used as a 10-fold dilution in distilled water)

(5)  Reagents for detecting non-radioactive marker

Blocking reagent: Phosphate bufferred

solution containing 2% BSA                   400 mℓ x 1

β-Galactosidase-streptavidin                 0.1 mℓ x 1

```
Washing solution: Phosphate buffer
solution                                    150 mℓ x 1
(used as a 10-fold dilution in distilled water)
Substrate solution: 4-methylumbelliferyl-
β-D-galactoside                             10 mℓ x 1
Diluting solution: Buffer solution
containing 1 mM MgCl₂                       100 mℓ x 1
(Substrate solution was used as 10-fold dilution in the
diluting solution)
```

The kit composed of the above components (1) - (5) was used in Example 3 and the bacterial species of the genus Streptococcus in the specimen were identified with high efficiency.

Beside the β-galactosidase-streptavidin in the kit, streptavidin or avidin labelled with peroxidase or alkali phosphatase may also be used.

Example 6

A kit was constructed as in Example 4 except that the marker biotin was replaced by Labezyme-peroxidase (POD). Stated more specifically, the constructed kit was the same what is described in Example 4 except for the use of the following labelling reagents and non-radioactive marker detecting agents.

| Labelling reagents | |
| --- | --- |
| Labezyme-POD reagents* (Labezyme-POD) | 200 μℓ x 1 |
| 25% Glutaraldehyde solution | 25 μℓ x 1 |

* Labezyme-POD set: Manufactured and sold by Wako Pure Chemical Industries, Ltd. and containing color forming reagents in addition to marker peroxidase (prepared by the method described in Japanese Patent Public Disclosure No. 60-501488).

| Non-radioactive marker detecting agents | |
| --- | --- |
| Substrate solution | 10 mℓ x 1 |
| Diluting solution (H₂O₂-containing buffer solution) | 100 mℓ x 1 |
| (Substrate solution was used as 10-fold dilution in the diluting solution) | |

Substrate solution containing one of the following substrates as a reagent capable of detecting and determining the enzymatic activity of peroxidase:

Tyramine hydrochloride[F]
p-cresol[F]
4-Hydroxyphenylacetic acid[F]
3-(4-Hydroxyphenyl)propionic acid[F]
2,2′-Azino-bis(3-ethylbenzothiazoline-6-sulfonic acid)
3,3′,5,5′-Tetramethylbenzidine
5-Aminosalicylic acid
(F: fluorescence emitting substrate)

Using the kit described above, a bacterial species of the genus Campylobacter was successfully identified.

Example 7

A kit was constructed as in Example 5 except that the marker biotin was replaced by Labezyme-peroxidase (POD). Stated more specifically, the constructed kit was the same what is described in Example 5 except for the use of the following labelling reagents and non-radioactive marker detecting agents.

| Labelling reagents | |
| --- | --- |
| Labezyme-POD reagents* (Labezyme-POD) | 200 $\mu\ell$ x 1 |
| 25% Glutaraldehyde solution | 25 $\mu\ell$ x 1 |

\* Labezyme-POD set: Manufactured and sold by Wako Pure Chemical Industries, Ltd. and containing color forming reagents in addition to marker peroxidase (prepared by the method described in Japanese Patent Public Disclosure No. 60-501488).

| Non-radioactive marker detecting agents | |
| --- | --- |
| Substrate solution | 10 m$\ell$ x 1 |
| Diluting solution (H$_2$O$_2$-containing buffer solution) | 100 m$\ell$ x 1 |
| (Substrate solution was used as 10-fold dilution in the diluting solution) | |

Substrate solution containing one of the following substrates as a reagent capable of detecting and determining the enzymatic activity of peroxidase:

Tyramine hydrochloride[F]
p-cresol[F]
4-Hydroxyphenylacetic acid[F]
3-(4-Hydroxyphenyl)propionic acid[F]
2,2′-Azino-bis(3-ethylbenzothiazoline-6-sulfonic acid)
3,3′,5,5′-Tetramethylbenzidine
5-Aminosalicylic acid

(F: fluorescence emitting substrate)

Using the kit described above, a bacterial species of the genus Streptococcus was successfully identified.

**Claims**

1. A method for identifying the species of a bacterium which comprises the steps of:

identifying a bacterial group to which the species of bacterium to be identified belongs by conventional techniques such as its physiological properties, biochemical properties, gram staining or morphological characteristics;

providing a plurality of containers each of which contains single-stranded chromosomal DNA extracted from a bacterial species immobilized in a solid phase, each container containing single-stranded chromosomal DNA extracted from a different bacterial species belonging to the bacterial group species

extracting chromosomal DNA from the bacterial to be identified, and either denaturing the extracted DNA to form single-stranded DNA which is then labelled with a non-radioactive marker or labelling the extracted DNA with a non-radioactive marker and then denaturing it to form single-stranded DNA;

adding the non-radiolabelled single-stranded DNA to the single-stranded DNA in said containers, performing hybridization and washing the containers to remove the excess labelled DNA; and

assaying the non-radioactive marker to determine the degree of hybridization and identifying the species of bacterium which has the highest degree of hybridization;

characterized in that the containers are individual wells of a microtiter plate on whose inner surfaces the single-stranded chromosomal DNAs are immobilized.

2. A method according to claim 1, wherein the non-radioactive marker is biotin or an enzyme.

3. A method according to claim 1, wherein the chromosomal DNA to be immobilized in a solid phase is one which has been extracted from a bacterium, cut to a fragment of an appropriate size and made single-stranded.

4. A kit for identifying the species of a bacterium according to claim 1, which comprises reagents for extracting DNA from bacterial cells to be assayed, a non-radioactive Labelling reagent for DNA, and containers each of which contains single-stranded chromosomal DNA extracted from a bacterial species immobilized in a solid phase, each container containing single-stranded DNA extracted from a different bacterial species belonging to the bacterial group;
   characterized in that the containers are individual wells of a microtiter plate on whose inner surfaces the single-stranded chromosomal DNAs are immobilized.

5. A kit according to claim 4, wherein the reagents are for extracting DNA from gram-negative bacteria.

6. A kit according to claim 4, wherein the reagents are for extracting DNA from gram-positive bacteria.

7. A kit according to claim 4, wherein the wells are detachable.

8. A kit according to claim 4, wherein the reagents are used in such small amounts that DNA can be extracted from cells whose quantity is equivalent to 1.5 ml of a cell suspension having a turbidity of McFarland No. 0.5-6.

**Patentansprüche**

1. Verfahren zum Identifizieren einer Bakterienspezies, umfassend die Schritte:
   Identifizieren einer Bakteriengruppe, zu der die zu identifizierende Bakterienspezies gehört, nach üblichen Techniken, wie ihren physiologischen Eigenschaften, biochemischen Eigenschaften, gram-Färbung oder morphologischen Eigenschaften;
   Bereitstellen einer Vielzahl von Behältern, von denen ein jeder einzelsträngige chromosomale DNA, extrahiert aus einer, in einer festen Phase immobilisierten Bakterienspezies, enthält, wobei jeder Behälter einzelsträngige chromosomale DNA enthält, die aus verschiedenen Bakterienspezies, zugehörig zu der Bakteriumgruppenspezies, extrahierte wurde;
   Extrahieren der chromosomalen DNA aus dem zu identifizieren Bakterium, und entweder Denaturieren der extrahierten DNA unter Bildung einzelsträngiger DNA, die anschließend mit einem nicht-radioaktiven Marker markiert wird, oder Markieren der extrahierten DNA mit einem nicht-radioaktiven Marker und anschließende Denaturierung unter Bildung einzelsträngiger DNA;
   Zugabe nicht-radioaktiv markierter einzelsträngiger DNA zu der einzelstängigen DNA in den Behältern, Durchführung der Hybridisierung und Waschen der Behälter unter Entfernen des Überschusses markierter DNA; und
   Testen der nicht-radioaktiven Marker unter Bestimmung des Ausmaßes der Hybridisierung und Identifikation der Bakterienspezies, welches das höchste Ausmaß an Hybridisierung zeigt;
   dadurch **gekennzeichnet,** daß die Behälter einzelne Wells in einer Mikrotiterplatte sind, auf deren inneren Oberflächen die einzelsträngigen chromosomalen DNAs immobilisiert sind.

2. Verfahren nach Anspruch 1, worin der nicht-radioaktive Marker Biotin oder ein Enzym ist.

3. Verfahren nach Anspruch 1, worin die in einer festen Phase zu immobilisierende chromosomale DNA eine ist, die aus einem Bakterium extrahiert wurde, in einem Fragment oder auf eine geeignete Größe geschnitten wurde, und einzelsträngig gemacht wurde.

4. Kit zur Identifizierung einer Bakterienspezies nach Anspruch 1, welches Reagenzien zur Extraktion von DNA aus zu testenden Bakterienzellen, ein nicht-radioaktives Markierungsreagens für DNA und Behälter enthält, von denen ein jeder einzelsträngige chromosomale DNA enthält, die aus der in einer festen Phase immobilisierten Bakterienspezies extrahiert wurde, wobei jeder Behälter einzelstränginge DNA, extrahiert aus verschiedenen Bakterienspezies, zugehörig zur bakteriellen Gruppe, enthält;
   dadurch **gekennzeichnet,** daß die Behälter einzelne Wells einer Mikrotiterplatte sind, auf deren inneren Oberflächen die einzelsträngigen chromosomalen DNAs immobilisiert sind.

**5.** Kit nach Anspruch 4, worin die Reagenzien zur Extraktion von DNA aus gram-negativen Bakterien dienen.

**6.** Kit nach Anspruch 4, worin die Reagenzien zur Extraktion von DNA aus gram-positiven Bakterien dienen.

**7.** Kit nach Anspruch 4, worin die Wells abnehmbar sind.

**8.** Kit nach Anspruch 4, worin die Reagenzien in solch kleinen Mengen verwendet werden, daß die DNA aus den Zellen extrahiert werden kann, deren Menge äquivalent zu 1,5 ml einer Zellsuspension mit einer McFarland-Turbidität von Nr. 0,5 bis 6 ist.

**Revendications**

**1.** Procédé d'identification de l'espèce d'une bactérie, comportant les étapes consistant à:

identifier un groupe bactérien auquel l'espèce de bactérie à identifier appartient, par des techniques classiques telles que ses propriétés physiologiques, ses propriétés biochimiques, une coloration de gram ou des caractéristiques morphologiques;

fournir plusieurs récipients contenant chacun de l'ADN chromosomique monobrin extrait d'une espèce bactérienne immobilisé dans une phase solide, chaque récipient contenant de l'ADN chromosomique monobrin extrait d'une espèce bactérienne différente appartenant à l'espèce du groupe de bactéries;

extraire de l'ADN chromosomique de la bactérie à identifier et, soit dénaturer l'ADN extrait pour former de l'ADN monobrin qui est alors marqué par un marqueur non radioactif, soit marquer l'ADN extrait par un marqueur non radioactif et le dénaturer ensuite pour former l'ADN monobrin;

ajouter l'ADN monobrin marqué par un marqueur non radioactif à l'ADN monobrin dans lesdits récipients, effectuer une hybridation et laver les récipients pour extraire l'ADN marqué en excès; et

analyser le marqueur non radioactif pour déterminer le degré d'hybridation et identifier l'espèce de bactérie qui présente le degré d'hybridation le plus élevé; caractérisé en ce que les récipients sont des cuvettes individuelles d'une plaque de micro-titration sur la surface interne desquelles les ADN chromosomiques monobrins sont immobilisés.

**2.** Procédé selon la revendication 1, dans lequel le marqueur non radioactif est la biotine ou une enzyme.

**3.** Procédé selon la revendication 1, dans lequel l'ADN chromosomique à immobiliser dans une phase solide est un ADN qui a été extrait d'une bactérie, coupé en un fragment de taille appropriée, et transformé en un ADN monobrin.

**4.** Module pour l'identification de l'espèce d'une bactérie selon la revendication 1, qui comporte des réactifs pour l'extraction d'ADN de cellules bactériennes à analyser, un réactif de marquage non radioactif de l'ADN, et des récipients qui contiennent chacun de l'ADN chromosomique monobrin extrait d'une espèce bactérienne, immobilisé dans une phase solide, chaque récipient contenant de l'ADN monobrin extrait d une espèce bactérienne différente appartenant au groupe de bactéries;

caractérisé en ce que les récipients sont des cuvettes individuelles d'une plaque de microtitration sur la surface interne desquelles les ADN chromosomiques monobrins sont immobilisés.

**5.** Module selon la revendication 4, dans lequel les réactifs sont destinés à extraire l'ADN provenant de bactéries gram-négatives.

**6.** Module selon la revendication 4, dans lequel les réactifs sont destinés à extraire l'ADN de bactéries gram-positives.

**7.** Module selon la revendication 4, dans lequel les cuvettes sont amovibles.

**8.** Module selon la revendication 4, dans lequel les réactifs sont utilisés en quantités suffisamment petites pour que l'ADN puisse être extrait de cellules dont la quantité est équivalente à 1,5 ml d'une suspension de cellules présentant un indice de turbidité de McFarland N° 0,5-6.

# Fig. 1

GROUPING OF CLINICAL BACTERIA AND THE TYPES OF KITS USED

EP 0 326 989 B1

# Fig. 2